Europäisches Patentamt

European Patent Office

Office européen des brevets

(11) Publication number: **0 291 636**
**A1**

# EUROPEAN PATENT APPLICATION

(21) Application number: 88102108.3

(22) Date of filing: 25.10.84

(51) Int. Cl.⁴ **A61K 39/395 , C12P 21/00 , C12N 5/00 , C12N 15/00**

(30) Priority: 07.11.83 US 549505
07.11.83 US 549506

(43) Date of publication of application:
23.11.88 Bulletin 88/47

(60) Publication number of the earlier application in accordance with Art.76 EPC: 0 141 783

(84) Designated Contracting States:
AT BE CH DE FR GB IT LI LU NL SE

(71) Applicant: THE WISTAR INSTITUTE
36th Street at Spruce
Philadelphia Pennsylvania 19104(US)

(72) Inventor: Koprowski, Hilary
334 Fairhill Road
Wynnewood Pennsylvania 19096(US)
Inventor: Reagan, Kevin
21 South Cliff Drive
Wycliffe Delaware 19809(US)
Inventor: Wiktor, Tadeusz
9 Downs Circle
Wynnewood Pennsylvania 19096(US)

(74) Representative: Norris, Allen Edwin et al
SANDOZ AG Patentabteilung Lichtstrasse 35
CH-4002 Basel(CH)

(54) Immune response to viruses induced by anti-idiotype antibodies.

(57) Anti-idiotype antibodies, their production and their use in inducing an immunological response to viruses.

EP 0 291 636 A1

# IMMUNE RESPONSE TO VIRUSES INDUCED BY ANTI-IDIOTYPE ANTIBODIES

## TECHNICAL FIELD

The present invention is directed to the induction of an immunological response to antigens and in particular viruses. More specifically, the present invention is directed to the use of anti-idiotype antibodies to induce such an immunological response, as well as the antibodies and cell lines that produce them.

## BACKGROUND OF THE INVENTION

The sequence of amino acids in the variable regions of both heavy ($V_H$) and light ($V_L$) chains of immunoglobulin (Ig) produces a conformation in the antigen binding site (ie parotope) allowing interaction of that antibody with a specific antigen. Injection of Ig into a heterologous host animal will give rise to anti-xenotypic (specific for species), anti-isotypic (specific for Ig class), and anti-idiotypic (specific for antibody variable region) antibodies. Two functional classes of anti-idiotypic antibodies can exist, one of which reacts with the parotape, and another which reacts with the $V_H$ and/or $V_L$ framework (framework determinants). See generally, Geha, (1981) N Engl J Med 305: 25-28; Jerne (1974) Ann Immunol (Paris) 125C: 373-389.

## SUMMARY OF THE INVENTION

It is an object of the present invention to provide a method of inducing an immunological response to viruses, for example rabies virus. It is also an object of the present invention to employ anti-idiotype antibodies to produce such an immunological response.

Yet another object of the present invention is to provide monoclonal anti-idiotypic antibodies, and immortal B lymphocyte sources for such antibodies, that are useful in the induction of immunological responses to viruses such as rabies virus.

These and other objects of the present invention are achieved by one or more of the following embodiments.

In one embodiment, the present invention provides a method of inducing an immunological response to an antigen selected from a virus comprising:

(a) providing an anti-idiotype antibody, an epitope identified by said anti-idiotype antibody being the parotope of an anti-virus antibody; and

(b) stimulating in a subject the production of anti-(anti-idiotype) antibody that identifies an epitope on a virus particle by administering said anti-idiotype antibody to said human.

The present invention also provides polyclonal anti-idiotype antibodies, and epitope identified by said anti-idiotype antibodies being the parotope of an anti-virus antibody, substantially free of anti-isotypic antibodies.

In another embodiment, the present invention provides an immortal B lymphocyte that produces an anti-idiotype antibody, an epitope identified by said anti-idiotype antibody being the parotope of an anti-virus antibody. The present invention also provides the monoclonal antibodies produced by the above immortal B lymphocyte substantially free of other antibodies.

Suitable viruses include influenza, herpes simplex, hepatitis and particularly rabies virus. On the administration of the anti-idiotype antibodies in accordance with the invention, the subject, in particular mammal, more particularly human, is stimulated to produce anti-(anti-idiotype) antibody that identifies an epitope on a virus particle.

## DETAILED DESCRIPTION OF THE INVENTION

The present invention provides a unique approach to viral immunisation. Traditionally anti-viral therapy has involved immunisation with conventional vaccines. Applicants, however, have discovered that an immunological response to viruses can be induced with an antibody that is anti-idiotypic to an antibody that recognises a virus antigen. The induction of this immunological response has utility as a therapeutic and preventative treatment.

Although applicants do not wish to be bound by any particular theory of operability, it is believed that the observed immunological response achieved by the present invention is attributed to an interaction between the anti-idiotype antibody molecules and the human patient's immune system. The idiotypic (ie variable) region of the anti-idiotype antibody molecule contains antigenic determinants (ie epitopes) seen as an antigen by the subject. This induces the production of anti-(anti-idiotype) antibodies by the subject. Within this set of anti-(anti-idiotype) antibodies are those that are directly complimentary to the parotope of the anti-idiotype antibody. It is further believed that the parotope of the anti-idiotype antibody presents an "internal" image of the virus epitope identified (ie selectively bound) by the idiotype antibody and, therefore, the anti-(anti-idiotype) antibodies will also bind the antigen on the virus particle. In effect, the present method induces an immunoligical response to the virus by presenting an antigen (the parotope of the anti-idiotype antibody) which is essentially indistinguishable from the virus antigen to a portion of the patient's resulting antibodies.

Surprisingly, the above method is an effective procedure for inducting an immunological response to a virus. Furthermore, it has several advantages over the more traditional approaches. First, much less foreign antibody need by adminstered to a patient. Second, the patient's anti-idiotype is beneficial rather than detrimental to the intended effect. Third, the patient's own antibodies are the anti-virus antibodies. Other advantages will be readily apparent to those skilled in the art.

The idiotype of an antibody is defined by individually distinctive antigenic determinants in the variable or idiotypic region of the antibody molecule. A portion of these idiotypic determinants will be on or closely associated with parotope of the antibody, while others will be in the framework of the variable region. While each antibody has its own idiotype, particular antibodies will be referred to below by the following terms, "Idiotype antibody" or "Id Ab" refers to an anti-virus antibody (ie the epitope identified by the idiotpye antibody is on a virus particle). "Anti-idiotype antibody" or "anti-Id Ab" refers toan antibody which identifies an epitope in the variable region of an idiotype antibody. A portion of such antibodies will identify an epitope that is the parotope of the idiotype antibody, thus presenting an "internal" image of the epitope identified by the idiotype antibody. "Anti-(anti-idiotype) antibody" or anti-(anti-Id) Ab" is an antibody that identifies an epitope in the variable region of the anti-idiotype antibody. A portion of the anti-(anti-idiotype) antibodies will identify an epitope that corresponds to (i) the parotope of the anti-idiotype antibody, and (ii) the epitope.

As stated below, the method of the present invention contemplates administering anti-idiotype antibody to a host. The anti-idiotype antibody is administered to the host in any physiologically suitable carrier (eg sterile, pyrogen-free physiological saline), the formulations of which are within the skill of the art. The selection of carrier is not critical and the antibody can be adminstered by any method that introduces the antibody into the circulatory system (eg intravenous, intramuscular or subcutaneous injection).

The host may be any mammal, most commonly a human, cat or dog. The amount of antibody administered to a host can vary widely, dependent, for example, upon the particular antibody employed and the patient inoculated. It is only necessary that sufficient anti-idiotype antibody be administered to stimulate the production of anti(anti-idiotype) antibodies by the patient's immune system. The amounts of antibody employed, however, need not be very great because only very small amounts are necesary to induce an immunological response. In many cases, a dosage of antibody within the range of a few micrograms to a few milligrams should be sufficient (eg about 50-200 $\mu$g to about 1-5 mg). The determination of an appropriate dosage is readily within the skill of the art.

A formulation containing an anti-idiotype antibody may be administered to a host wherein the parotope of the anti-idiotype antibody is an internal image of a virus antigen. Such an antibody recognises an epitope that is the parotope of the corresponding idiotype antibody. Anti-idiotype antibodies, which present internal images of the tumour antigen, can be distinguished from anti-idiotype antibodies that recognise framework determinants in the variable region of the idiotype antibody by any of several methods. One method of identifiying the desired anti-idiotype antibodies is a competitive binding assay between the virus antigen (or hapten if available), the idiotype antibody and the anti-idiotype antibody. If the viral antigen blocks binding of the anti-idiotype antibody to the idiotype antibody, the epitope identified by the anti-idiotype antibody is closely associated with the idiotype antibody's parotope. Another test is to determine if anti-sera to the antiidiotype antibody is also anti-virus. These and other methods of identifying the appropriate anti-idiotype antibody are within the skill of the art. In the formation administered to a host, the inclusion of anti-idiotype antibodies directed to framework determinants along with the subclass directed to the idiotype antibody's parotope is acceptable. It is only necessary that the formulation contain the subclass directed to the idiotype antibody's parotope.

The anti-idiotype antibody employed can be homologous or heterologous to the host. The preferred antibody for a human is however a human antibody to minimise immunological response to the constant

3

region of the antibody molecule. However, since relatively small doses of anti-idiotype antibody are required in the present invention, heterologous antibody can be employed (eg mouse, rat, goat, rabbit, etc). In the absence of any serious reaction to heterologous anti-idiotype antibody, however, such antibody may be preferred due to ease and cost of preparation. Furthermore, polyclonal anti-idiotype antibodies can be employed as well as monoclonal anti-idiotype antibodies.

Polyclonal anti-idiotype antibody can be prepared by conventional methods known in the art. For example, polyclonal anti-Id Ab can be produced by immunising an animal with a monoclonal anti-virus antibody (ie Id Ab). The immunised animal will produce anti-Id Ab. A subclass of this anti-idiotype antibody in the anti-sera will identify an epitope that is the parotope of the anti-virus antibody. Anti-sera collected from the animal can be purified, for example, by sequential absorbtion with (i) an immobilised antibody of the same isotype as the monoclonal Id Ab, but different idiotype, to remove anti-isotypic antibodies from the anti-sera. and (ii) the immobilised monolonal Id Ab to remove the anti-Id Ab, a subclass of which will present internal images of the virus antigen. The anti-Id Ab can then be eluted from the bound mono clonal anti-virus antibody to provide a solution substantially free of anti-isotype antibodies. This solution can then be tested for the presence of the anti-Id Ab that identifies the parotope of the Id Ab.

Monoclonal anti-idiotype antibodies substantially free of other antibodies can be isolated from the supernatant of substantially pure cultures of immortal B lymphocytes. The term "immortal B lymphocyte" encompasses any relatively stable, continuous antibody-producing cell that can be maintained in culture for several months (preferably indefinitely), such as hybridomas (somatic cell hybrids of normal and malignant lymphocytes) and normal lymphocytes transformed by virus (eg Epstein-Barr virus) or oncogenic DNA. The production of immortal B lymphocytes from normal B lymphocytes that produce anti-idiotype antibody is within the skill of the art. See, eg Monoclonal Antibodies (R H Kennett, T J McKearn & K B Bechtol 1980); M Schreier et al Hybridoma Techniques (Cold Spring Harbour Laboratory 1980); Monoclonal Antibodies and T-Cell Hybridomas (G J Hammerling, U Hammerling & J F Kearney 1981); Kozbor et al (1982) PNAS 79: 6651-6655; Jonak et al (1983) Hybridoma 2: 124; Monoclonal Antibodies and Functional Cell Lines (R H Kennett, K B Bechtol & T J McKearn 1983); Kozbor et al (1983) Immunology Today 4: 72-79.

Normal B lymphocytes producing anti-Id Ab and suitable for the production of an immortal B lymphocyte can be provided by various methods within the skill of the art. For example, an animal, such as a rat or mouse, can be immunised with a monoclonal anti-virus antibody and B lymphocytes producing anti-Id Ab recovered from the animal's spleen. Human B lymphocytes producing anti-Id Ab can be obtained by immunising a patient with monoclonal anti-virus antibody, collecting peripheral blood lymphocytes from the patient, and then inducing in vitro the growth of B lymphocytes producing anti-Id Ab by stimulating the culture with the monoclonal anti-virus antibody. See, eg DeFreites et al (1982) PNAS 79:6646-6650. The animal or human B lymphocytes producing anti-Id Ab can thus be recovered and immortalised by those of skill in the art. Of course it is understood that those lymphocytes producing anti-Id Ab that present internal images of the virus particle should be distinguished from B lymphocytes producing anti-Id Ab directed to framework determinants in the idiotypic region.

If desired the immune response produced in the host mammal to a virus can be further heightened by immunisation with a conventional viral vaccine in addition to the administration of anti-Id Ab as described above. After the production of anti-(anti-Id) Ab is stimulated in the host mammal by administering anti-Id Ab (eg, about 2 weeks post-administration), the host is then given one inoculation of a virus vaccine employing conventional techniques known in the art (eg, killed virus vaccines such as HDC rabies vaccine). See, Plotkin et al (1976) Am J Epidemiology 103: 75-80; Wiktor et al Rabies Vaccines For Human Use Vol 40; 3-9 (1978). The types of vaccine and protocols for their administration are within the skill of the art.

The following examples are illustrative of the present invention and are not intended to limit its scope.

## Preparation of Anti-Idiotype Antibody

The ability of anti-idiotype antibody (anti-Id Ab) made against various monoclonal antibodies (mAb) to reconstruct the epitopes of rabies virus glycoprotein (G) was studied. This G is responsible for many of the important biological properties of rabies virus, including the induction of the virus-neutralizing antibodies (VNA). Precisely which portion of the G is reponsible for this function is not known at present. A functional epitope mat for challenge virus standard (CVS) strain of rabies virus G suggesting the existance of at least three major antigenic sites for type-specific VNA has been described in Lafon et al., (1983) J. Gen. Virol. 64:843-851.

Five anti-G mAb were selected from a large panel of hybridomas on the basis of their isotype (enabling purification by protein A-Sepharose chromotography) and binding site on the rabies virus G as defined by the functional epitope mat. Anti-rabies virus G mAb's 509-6, 101-1, 507-1 and 719-3 have been described previously. Lafon et al., supra; Wiktor et al., (1980) J. Exp. Med. 152:99-112. These mAb, which strongly neutralize rabies virus infectivity, have the following characteristics: 509-6 (epitope map site I; IgG2a), 101-1 (epitope map site IIb; IgG2a), 507-1 (epitope map site IIIb; IgG1), and 719-3 (epitope mat site IIc; IgG2a). Anti-G mAb 1104-2 (IgG1) was obtained from an additional fusion of splenocytes from rabies virus immunized BALB/c mice with the 653 variant of P3x63Ag8 mouse myeloma cells, Kearney et al., (1979) J. Immunol. 123:1548-1550, as described in Wiktor et al., (1978) Proc. Natl. Acad. Sci. USA 75:3938-3942. The anti-G-secreting hybridoma cells were selected, cloned by limiting dilution, and ascites fluids prepared as also described in Wiktor et al., (1978), supra. The 1104-2 mAb was selected because it demonstrated excellent binding to ERA virus, but poor neutralization. Anti-rabies virus nucleocapsid mAb 515-3 (IgG2a) and 389-1 (IgG1) were isolated by similar techniques from Kelev virus-immunized BALB/c mice.

The stocks of ERA or CVS strains of rabies virus employed were propagated in BHK-21 cells by standard methods. See Wiktor et al., in Laboratory Techniques in Rabies, pp. 101-123 (M. Kaplan & H. Koprowski 3rd ed. 1973). Rabies antigenic variants, ERA RV194-2 and RV509-6, have been described and represent viruses which are resistant by neutralization by anti-G mAb 194-2 and 509-6, respectively. See Dietzschold et al., (1983) Proc. Natl. Acad. Sci. USA 80: 70-74. Rabies soluble glycoprotein ($G_s$) was purified from virion-depleted culture fluids by immunoadsorbent chromotography as described in Dietzschold et al., (1983) Virology 124: 330-337.

All anti-G mAb were purified from ascites fluids. Antibodies of the IgG2a isotype were diluted appropximately 1:30 with Britton-Robinson buffer (BRB) at pH 8.0. See Gerhard et al., in Monoclonal Antibodies, pp. 317-333 (R. Kennett, T. McKearn & K. Bechtol 1980). The mAb in BRB was passed through a Nalgene 0.45 u filter, and then over a protein A-Sepharose 4B absorption column (Pharmacia, Piscataway, N.J.). The column was washed with 30 ml of BRB, pH 8.0, before antibody was eluted with BRB at pH 3.0. Anti-G mAb of IgG1 isotype were first precipitated with sodium sulfate at a final concentration of 18% (w/v). The Ig fraction was dissolved and dialyzed in BRB, pH 8.0, and then passed over the protein A-Sepharose column as before. Ig eluted from the column was detected by radioimmunoassay (RIA) using ERA virus as antigen. See, Dietzschold et al., (1982) J. Virol. 44: 595-602. Antibodies were concentrated by vacuum dialysis against phosphate-buffered saline, pH 7.4 (PBS). Protein concentration was determined using bovine serum albumin as a standard. See Bramhall et al., (1969) Anal Biochem. 31: 146-148.

Anti-Id Ab were prepared as described in Staudt et al., (1983) J. Exp. Med. 157: 687-704. Briefly, femal New Zeland white rabbits were injected subcutaneously in multiple sites along the mammary chain with 300 ug of protein A-Sepharose-purified mAb emulsified in Freund's complete adjuvant (FCA). Two intramuscular boosters of 100 ug of antibody in PBS were given on days 7 and 30, and sera are collected 10 days later. Each anti-idiotypic anti-serum was made specific for idiotype regions by passage over a Sepharose 4B column to which either mAb 515-3 (IgG2a) or 389-1 (IgG1) were coupled to remove anti-idiotype Ab. The effluent from such columns contained the antibodies reactive with the idiotype determinants. Antibodies to constant regions were eluted from the mAb 515-3 and 389-1 immunoadsorbent columns with 0.1 diethylamine, pH 11.5. IgG from each anti-Id Ab was isolated by protein A-Sepharose chromotography as above. The reactivity of the column effluent with non-idiotype determinants was neglegible.


Characterization of Anti-Idiotype Antibodies


The specificity of the anti-Id Ab prepared above and the existence of cross-reactive idiotypes among the various anti-G mAb was determined for each anti-Id Ab preparation in a RIA.

A solid phase RIA was used to measure the bindings of anti-Id Ab to fixed mAb. Individual mAb were diluted depending on the ascites fluid concentration in carbonate-bicarbonate buffer, pH 8.9, as follows: 509-6 (1:3,000), 101-1 (1:6,000), 719-3 (1:6,000), 507-1 (1:6,000), 1104-2 (1:16,000). Then 25 ul of each was added to wells of polyvinyl microtiter plates (Dynatech Laboratories). These antibodies were allowed to dry to the wells by overnight incubation at 37°C. Free binding sites on the wells were blocked for at least 1 hour with 10% agamma horse serum (GIBCO Laboratories) in PBS with 0.08% sodium azide (PBSN). Twenty-five microliters of dilution of anti-Id Ab was added, and after 1 hour at room temperature, the plates were washed extensively. Bound anti-Id Ab was detected by adding 25 ul (30,000 cpm) of $^{125}$I goat anti-rabbit IgG (Cappel Laboratories) labeled by the iodogen method and incubating for an additional hour at room temperature. See Markwell et al., (1978) Biochemistry 17: 4807-4817. All dilutions of anti-Id Ab or radiolabeled probe were made in 10% agamma horse serum in PBSN. Plates were washed free of unbound probe and radioactivity bound to individual wells was measured in a gamma counter.

The results of titrating each anti-Id Ab preparation against homologous and heterologous mAb demonstrated that each anti-Id Ab was specified for its homologous Id mAb.

## Determination of Anti-Idiotype Antibodies Directed to the Parotope of the Idiotype Antibody

A competition RIA was devised to test the ability of rabies virus G to prevent the interaction between Id Ab and anti-Id Ab. See, Chaflin et al., (1974) J. Immunol. 112:1747-1756; Sher et al., (1972) J. Immunol. 109:176-178.

The rabies $G_s$ was used as the competing antigen. Pretitrated levels of mAb were inculbated with serial two-fold dilutions of $G_s$ for 1 hour prior to the addition of a standardized anti-Id Ab dilution. The amount of bound anti-sera was determined by the binding of a $^{125}I$-labeled goat anti-rabbit antibody probe. The binding of three of the five anti-Id Ab to their corresponding anti-G mAb (509-6, 507-1 and 1104-2) was inhibited by $G_s$. Maximum inhibition varied from 20-50% with as much as 6 ug/ml $G_s$, but at least a 15% reduction was observed with as little as 0.75 ug/ml $G_s$. The inability to totally inhibit binding of anti-Id Ab was an indication that both framework and parotope site specificities were present in the three polyclonal anti-Id sera. This was confirmed by a Western blot analysis of anti-Id Ab reactivity with mAb which had been resolved by sodium dodecyl sulfate-polyacrylamide gel electrophoresis (SDS-PAGE) under reducing and non-reducing conditions. Since the antigen-combining site is eliminated by reduction of mAb, the reactivity of anti-Id Ab with reduced mAb showed a specificity for framework determinants. The absence of any significant binding inhibition by $G_s$ for anti-Id 101-1 Ab or anti-Id 719-3 Ab, suggests a minor or absent population of parotope specificities in these sera.

## Preparation of Anti-(Anti-Idiotype)Antibody

The following examples demonstrates that anti-Id Ab reactive with the antigen-combining site of anti-G mAb contains a subpopulation which mimics the viral epitope recognized by these anti-G mAb and can induce an immunological response to G.

ICR mice in groups of 4 were inoculated subcutaneously with 40 ug/mouse of protein A-Sepharose-purifed anti-Id IgG emulsified in FCA. Subcutaneous booster inoculations were administered on day 7 and 32; each with 40 ug of anti-Id IgG in Freund's incomplete adjuvant. Five days following the last booster inoculation, animals were bled via the retro-orbital plexus and pooled sera checked for rabies virus-neutralizaing antibodiy (VNA). As a control, another group of mice received an immunization of protein A-Sepharose-purified normal rabbit IgG.

The levels of VNA in immunized mice were determined by a modification of the rapid fluorescence focus inhibition test. See Smith et al, in Laboratory Techniques in Rabies, pp. 354-357 (M. Kaplan & H. Koprowski 3rd ed. 1973). Serial two-fold dilutions of mouse serum were prepared in Microtiter II plates (Falcon Plastics) (50 ul/well) and incubated for 1 hour at 37°C with an equal volume of virus, containing $10^4$ PFU/50 ul. Following incubation, 50 ul of freshly trypsinized BHK-21 cells (2 x $10^6$ cells/ml) were added to each well, mixed, and 10 ul aliquots of the serium-virus-cell mixture were transferred (in duplicate) into wells of Terasaki plates (Falcon Plastics). After 20 hour incubation, plates were first rinsed with PBS and then with 80% (v/v) acetone in distilled water, and fixed for 30 min in 80% acetone at room temperature. Plates were dried and cells were stained for 30 min at 37°C with 5 ul/well of fluorescein-conjugated anti-rabies nucleocapsid antibody of rabbit origin. See Wiktor, (1974) Symp. Series Immunobiol. Standard 21: 102-118. The control wells (containing virus but no antibody) showed approximately 40% of cells containing rabies virus-specific inclusions. The end point of virus neutralization was defined as the reciprocal of the highest serum dilution capable of reducing the number or rabies virus-infected cells by 50%.

The results of the rapid fluorescent forcus inhibition test is shown in the table below. Significant VNA titers were generated against ERA strain rabies virus in mice immunized with anti-Id 509-6 Ab, and with anti-Id 1104-2 Ab. The three other anti-(anti-Id) Ab failed to neutralize ERA virus. Control experiments showed that mouse anti-(normal rabbit IgG) Ab as well as the anti-Id Ab used for immunization, had no rabies virus neutralizing activity. Furthermore, preincubation of anti-(anti-Id 509-6) sera with anti-Id 509-6 Ab removed the neutralizing activity. Preincubation with normal rabbit serum, however, did not.

In order to test the specificity of the VNA generated, other rabies viruses were used in the neutralization assay. The CVS strain of rabies virus bound both mAb 509-6 and 1104-2 (data not shown), and the Table shows that CVS was neutralized by both anti-(anti-Id 509-6) sera and anti-(anti-Id 1104-2) sera. On the other hand, a variant of ERA virus, RV 509-6, which possessed a mutation in the 509-6 epitope resulting in the loss of binding or neutralizing activity by mAb 509-6, see Laton et al., (1983) J. Gen. Virol. 64: 843-851, was not neutralized by anti-(anti-Id 509-6) sera. Another neutralization-resistant variant, RV 194-2, was effectively neutralized by anti-(anti-Id 509-6) sera. Previous results demonstrated that the antigenic sites recognized by anti-G mAb 509-6 and 194-2 are totally independent. See Laton et al., supra. These data show that anti-(anti-Id 509-6) sera reacts solely with the epitope which is recognized by anti-G mAb 509-6, and thus this epitope had been simulated by anti-Id 509-6 Ab.

### Neutralization of Rabies Virus Strains by Anti-(anti-Id) Serum†

| Mouse antiserum to: | ERA | | | CVS |
|---|---|---|---|---|
| | Parent | RV 509-6 | RV 194-2 | Parent |
| Normal rabbit IgG | 4 | 4 | 4 | 4 |
| anti-Id 509-6 | 32 | 4 | 64 | 64 |
| anti-Id 101-1 | 4 | ND* | ND | ND |
| anti-Id 179-3 | 4 | ND | ND | ND |
| anti-Id 507-1 | 4 | ND | ND | ND |
| anti-Id 1104-2 | 128 | 4 | 128 | 64 |
| RIG** | 16 | 16 | 32 | 32 |

† The reciprocal of the highest dilution of serum capable of reducing the number of infected cells by 50% was taken as the neutralization titer.

* Not done.

** Human anti-rabies Ig was diluted to 0.2 international units per ml, and serial two-fold dilutions incubated with rabies virus.

The above examples were presented for illustrative purposes only and are not intended to limit the invention which is defined solely by the claims.

Claims

1 A composition for inducing an immunological response to a virus comprising anti-idiotype antibodies which identify an epitope which is the parotope of an anti-virus antibody.

2 A composition according to claim 1 wherein the anti-idiotype antibodies are monoclonal.

3 A composition according to claim 1 wherein the anti-idiotype antibodies are polyclonal.

4 An immortal B lymphocyte that produces anti-idiotype antibodies which identify an epitope which is the parotope of an anti-virus antibody.

5 An immortal B lymphocyte according to claim 4 which is hybridoma.

6 Monoclonal antibodies obtainable from an immortal B lymphocyte according to claim 4 or 5 and substantially free of other antibodies.

7 Polyclonal anti-idiotype antibodies which identify an epitope which is the parotope of an anti-virus antibody, and which are substantially free of anti-isotype antibodies.

0 291 636

8 A method for the production of anti-idiotype antibodies which identify an epitope which is the parotope of an anti-virus antibody, which comprises administering an anti-virus antibody to a heterologous host, and isolating and purifying the desired anti-idiotype antibodies from the serum of the host.

8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF IMMUNOLOGY, vol. 131, no. 5, November 1983, pages 2539-2541, The American Association of Immunologists, US; R.S. KAUFFMAN et al.: "Cell receptors for the mammalian reovirus. II. monoclonal anti-idiotypic blocks viral binding to cells" * Whole article * --- | 1-2,4-6,8 | A 61 K  39/395<br>C 12 P  21/00<br>C 12 N  5/00<br>C 12 N  15/00 |
| X | JOURNAL OF IMMUNOLOGY, vol. 131, no. 5, November 1983, pages 2533-2538, The American Association of Immunologists, US; J.H. NOSEWORTHY et al.: "Cell receptors for the mammalian reovirus. I. syngeneic monoclonal anti-idiotypic antibody identifies a cell surface receptor for reovirus" * Abstract: "Materials and Methods" * --- | 1-2,4-6,8 | |
| X | SCIENCE, vol. 221, 26th August 1983, pages 853-855; R.C. KENNEDY et al.: "Immune response to hepatitis B surface antigen: enhancement by priop injection of antibodies to the idiotype" * Whole article * --- | 1,3,7-8 | **TECHNICAL FIELDS SEARCHED (Int. Cl.4)**<br><br>A 61 K<br>C 12 P |
| A | IMMUNOLOGICAL REVIEW, vol. 52, 1980, pages 75-88, Munksgaard, Copenhagen, DK; G. KELSOE et al.: "Control of idiotope expression by monoclonal anti-idiotope antibodies" --- -/- | | |

The present search report has been drawn up for all claims

| Place of search | Date of completion of the search | Examiner |
|---|---|---|
| THE HAGUE | 12-09-1988 | ALVAREZ Y ALVAREZ C. |

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | CLINICAL IMMUNOLOGY AND IMMUNOPATHOLOGY, vol. 21, 1981, pages 397-406, Academic Press, Inc.; A. NISONOFF et al.: "Implications of the presence of an internal image of the antigen in anti-idiotypic antibodies: possible application to vaccine production" --- | | |
| A | INFECTION AND IMMUNITY, vol. 34, no. 1, October 1981, pages 200-207; J. GHEUENS et al.: "Idiotypes and biological activity of murine monoclonal antibodies against the hemagglutinin of measles virus" --- | | |
| A | JOURNAL OF EXPERIMENTAL MEDICINE, vol. 155, April 1982, pages 1108-1119; D.L. SACKS et al.: "Immunization of mice against african trypanosomiasis using anti-idiotypic antibodies" --- | | TECHNICAL FIELDS SEARCHED (Int. Cl.4) |
| X,P | JOURNAL OF VIROLOGY, vol. 48, no. 3, December 1983, pages 660-666, American Society for Microbiology; K.J. REAGAN et al.: "Anti-idiotypic antibodies induce neutralizing antibodies to rabies virus glycoprotein" * Whole article * --- | 1-8 | |
| E | EP-A-0 110 706  (BAYLOR COLLEGE OF MEDICINE) * Claims 8-11; pages 1-15,28-29 * ----- | 1,3,7-8 | |

The present search report has been drawn up for all claims

| Place of search | Date of completion of the search | Examiner |
|---|---|---|
| THE HAGUE | 12-09-1988 | ALVAREZ Y ALVAREZ C. |

EPO FORM 1503 03.82 (P0401)